Europäisches Patentamt

European Patent Office

Office européen des brevets

(11) Veröffentlichungsnummer: **0 232 876**
**A2**

(12) **EUROPÄISCHE PATENTANMELDUNG**

(21) Anmeldenummer: 87101722.4

(22) Anmeldetag: 07.02.87

(51) Int. Cl.4: **C12P 7/06** , C12N 1/18 ,
//C12R1:865

(30) Priorität: 13.02.86 DE 3604484

(43) Veröffentlichungstag der Anmeldung:
19.08.87 Patentblatt 87/34

(84) Benannte Vertragsstaaten:
AT BE CH DE ES FR GB GR IT LI LU NL SE

(71) Anmelder: PFEIFER & LANGEN
Linnicher Strasse 48
D-5000 Köln 41(DE)

(72) Erfinder: Swyzen, Werner, Dipl.-Ing.
Grüner Weg 2
D-5177 Titz-Ameln(DE)

(74) Vertreter: Werner, Hans-Karsten, Dr. et al
Deichmannhaus am Hauptbahnhof
D-5000 Köln 1(DE)

(54) Verfahren zur alkoholischen Gärung durch Hefen, neue Hefen und ihre Verwendung.

(57) Bei der üblichen alkoholischen Gärung durch Hefen von assimilierbaren Zuckern bei Temperaturen zwischen 20 und 40°C in an sich bekannter Weise wird der pH-Wert zwischen 2,7 und 4,0 eingestellt und eine Hefe, beispielsweise der Hinterlegungsnummer DSM 3649, eingesetzt. Es können auch infizierte und nicht pasteurisierte Rohstoffe eingesetzt werden. Die Hefe arbeitet in einem pH-Bereich, in dem das Wachstum von Milchsäurebakterien unterdrückt ist.

EP 0 232 876 A2

## Verfahren zur alkoholischen Gärung durch Hefen, neue Hefen und ihre Verwendung

Gegenstand der vorliegenden Erfindung ist ein Verfahren zur alkoholischen Gärung durch Hefen von assimilierbaren Zuckern bei Temperaturen zwischen 20 und 40°C in an sich bekannter Weise. Derartige alkoholische Gärungen werden im allgemeinen im pH-Bereich zwischen 4,5 und 6 und bei Temperaturen zwischen 6 und 38°C durchgeführt. Unter diesen Bedingungen können auf den verwendeten Nährböden oder Substraten auch andere Mikroorganismen gedeihen, insbesondere Milchsäurebakterien. Es muß daher in der Praxis und insbesondere bei kontinuierlichen Verfahren steril gearbeitet werden. Trotzdem sind Fremdinfektionen noch immer das größte Problem aller kontinuierlichen Gärungsverfahren. Auch alle Versuche, die alkoholische Gärung nicht mit Hefen, sondern mit Kulturen von Zymomonas durchzuführen, haben in der Praxis noch nicht zu dem gewünschten Erfolg geführt, da immer wieder Fremdinfektionen auftreten und damit das Gesamtverfahren schwerwiegend stören.

Es besteht somit noch immer die Aufgabe, die alkoholische Gärung durch Mikroorganismen so durchzuführen, daß auch kontinuierliche Produktionsverfahren nicht durch Fremdinfektionen gestört werden.

Es wurde jetzt überraschenderweise gefunden, daß es Hefen gibt, die im pH-Bereich zwischen 2,7 und 4,0, vorzugsweise zwischen 3,2 und 3,8, wachsen und die assimilierbaren Zucker alkoholisch vergären. In diesem pH-Bereich kommt das Wachstum aller bisher bekannten Hefen völlig zum Stillstand. Auch die alkoholische Gärung ist bei diesen pH-Werten kaum noch zu beobachten. Diese niedrigen pH-Werte sind sogar in der Lage, das Wachstum von Milchsäurebakterien zum Stillstand zu bringen. Es ist daher erfindungsgemäß erstmals möglich, die alkoholische Gärung durch Hefen von assimilierbaren Zuckern bei Temperaturen zwischen 20 und 40°C in an sich bekannter Weise durchzuführen, dadurch, daß bei pH-Werten zwischen 2,7 und 4,0 eine Hefe der Hinterlegungsnummer DSM 3649 gezüchtet wird und das gebildete Ethanol in an sich bekannter Weise abgetrennt wird.

Dieses Verfahren kann problemlos kontinuierlich durchgeführt werden, da Fremdinfektionen nicht zu Störungen führen. Es können auch stark infizierte Rohstoffe ohne vorherige Pasteurisierung eingesetzt werden. Auch der Zusatz von Antibiotika oder anderen wachstumshemmenden Substanzen ist nicht notwendig. Es können daher erfindungsgemäß enzymatisch aufgeschlossene Stärken, Reststärken, Rübensäfte und Melassen problemlos mit Hilfe der neuen Hefen alkoholisch vergoren werden. Auch verzuckertes Holz und verzuckerte Cellulose kann erfindungsgemäß vergoren werden.

Optimale Wachstums-und Vergärungsbedingungen liegen im Temperaturbereich zwischen 30 und 35°C. Der pH-Bereich von 2,7 bis 4,0 kann in üblicher Weise eingestellt werden, beispielsweise durch Zugabe von Milchsäure, Salzsäure oder Schwefelsäure. Sofern zuvor eine Milchsäuregärung stattgefunden hat, kommt diese im allgemeinen bereits in dem erfindungsgemäß eingesetzten pH-Bereich zum Stillstand. Ein derartiges Ausgangsmaterial kann ohne Pasteurisierung mit der erfindungsgemäßen Hefe angeimpft und dann alkoholisch vergoren werden.

Die erfindungsgemäßen Hefen, beispielsweise gemäß Hinterlegungsnummer DSM 3649, können dadurch gewonnen und isoliert werden, daß man beispielsweise enzymatisch aufgeschlossene Stärken auf einen pH-Wert von 2,5 bis 2,8 einstellt und über längere Zeit in geschlossenen Gefäßen stehen läßt. Es wird dann gelegentlich beobachtet, daß sich der Druck in den Gefäßen erhöht und eine alkoholische Gärung einsetzt. Derartige Proben läßt man noch einige Zeit weiterwachsen und isoliert aus ihnen darin die neuen Hefen.

Diese neuen Hefen, insbesondere die Hefe der Hinterlegungsnummer DSM 3649, können somit ausgezeichnet für die alkoholische Gärung von assimilierbaren Zuckern verwendet werden, und zwar in einem pH-Bereich von 2,7 bis 4,0, vorzugsweise 3,2 bis 3,8. Diese Verfahrensbedingungen sind gegenüber den bisher üblichen aus den oben genannten Gründen außerordentlich vorteilhaft.

In den nachfolgenden Beispielen ist die Erfindung näher erläutert.

### 1. Gewinnung der Hefe

Eine mit Alpha-amylase und Amyloglucosidase aufgeschlossene Stärke wurde ohne Sterilisation mit Salzsäure auf pH 2,5 bis 2,8 eingestellt und mehrere Wochen stehengelassen. Nach dieser Zeit wurde ein Druckanstieg in dem verschlossenen Gefäß beobachtet. Eine weitere Untersuchung ergab, daß sich trotz des niedrigen pH-Wertes eine Hefe vermehrt hatte und alkoholische Gärung eingeleitet hatte. Die Hefe wurde aus diesem Ansatz in an sich bekannter Weise isoliert und mehrfach durch Überimpfen auf Reinheit und Stabilität geprüft.

Es wurde gefunden, daß sie bei 4 bis 5°C auf Schrägagar Oxytetracycline-Glucose-Yeast-Extract der Firma Oxoid mindestens eine Woche haltbar ist. Dieser Agar besteht aus 5 g/l Yeast Extract, 20 g/l Dextrose, 0,0001 g/l Biotin, 12 g/l Agar und 0,1 g/l Oxytetracyclin. Die Überprüfung der Lebensfähigkeit ist möglich auf einem Flüssigmedium in gleicher Zusammensetzung wie der Schrägagar, jedoch ohne Agar, oder einer Lösung aus 100 g/l Glucose, 0,375 g/l $MgSO_4$ $\times$ 7 $H_2O$, 0,75 g/l $KH_2PO_4$, 0,75 g/l $(NH_4)_2SO_4$ und 10,0 g/l Yeast Extract. Hierbei wird bei Temperaturen von ca. 30°C gearbeitet. Die Überprüfung erfordert ca. 24 h.

Es wurde festgestellt, daß es sich um Hefe handelt, die runde, leicht ovale Zellen mit 4 bis 6 nm Durchmesser aufweist. Sie benötigt Sauerstoff zum Wachsen. Sie metabolisiert Fructose, Glucose, Maltose und Saccharose. Sie wurde bei der Deutschen Sammlung von Mikroorganismen hinterlegt und hat die Hinterlegungsnummer DSM 3649 bekommen.

### 2. Alkoholische Gärung

Abfallstärke aus der Glucoseherstellung aus Weizenmehl als Rohstoff wird konzentriert auf ca. 15 % Trockensubstanz. Dieses Material wird mit Alpha-amylase verflüssigt und mit Amyloglucosidase verzuckert. Die Verflüssigungstemperatur beträgt 70°C, die Reaktionszeit 2 h. Die Verzuckerungstemperatur beträgt 55 bis 60°C, die Reaktionszeit 36 bis 48 h. Die so entstandene Glucoselösung wird kontinuierlich in einen Fermenter gepumpt, in dem der pH-Wert auf 3,3 bis 3,6 eingestellt ist. Die Temperatur beträgt 35°C. Die Aufenthaltszeit im Fermenter beträgt ca. 12 h. Die Glucosekonzentration im Zulauf beträgt ca. 100 bis 120 g/l, die Alkoholkonzentration im Fermenter liegt zwischen 50 und 60 g/l.

Der Fermenter wird schwach belüftet mit einer Belüftungsrate von 0,2 bis 0,8 m³ Luft pro m³ Fermenterraum und Stunde. In dem Fermenter befindet sich eine wachsende Kultur der Hefe DSM 3649. Bei Beginn des Verfahrens wird der Fermenter in üblicher Weise über Stellhefe angeimpft. Während der Gärung steigt der pH-Wert langsam an. In gleichem Maße steigt die Konzentration an Milchsäure. Durch Ansäuern bzw. Absenken des pH-Wertes auf einen tieferen Wert wird die Milchsäureproduktion wieder gestoppt, ohne daß die Hefe ihr Wachstum und ihre alkoholische Gärung einstellt.

Der Auslauf aus dem Fermenter kann ohne Abtrennung der Hefe der Destillation zugeführt werden.

Es ist möglich, die Hefe von der vergorenen Maische abzutrennen und gewünschtenfalls in den Fermenter zurückzuführen. Hierdurch kommt es zu einer erhöhten Konzentration der Biomasse und einer erhöhten Produktionsrate pro Raumeinheit Fermenter. Weiterhin kann diese Hefe auch in lebensfähiger Form abgetrennt und zur Animpfung weiterer Fermenter oder als Biomasse verwendet werden.

Eine genauere Untersuchung des Stammes DSM 3649 durch die Deutsche Sammlung von Mikroorganismen hat ergeben, daß es sich um Saccharomyces cerevisiae Meyen ex Hansen 1883 handelt. Eine Untersuchung von verschiedenen Hefestämmen Saccharomyces cerevisiae und insbesondere Saccharomyces cerevisiae Meyen ex Hansen 1883 hat jedoch ergeben, daß diese die übliche Säureempfindlichkeit beim Wachstum und der Ethanolerzeugung zeigen wie die bekannten Hefen.

Bei dem Stamm DSM 3649 handelt es sich somit offensichtlich um eine Mutante, die auch bei wesentlich niedrigerem pH-Wert vermehrungsfähig ist und Ethanol erzeugen kann.

Das erfindungsgemäße Verfahren hat sich inzwischen im technischen Maßstab bewährt und wurde jeweils mehrere Monate hintereinander störungsfrei durchgeführt.

### Ansprüche

1. Verfahren zur alkoholischen Gärung durch Hefen von assimilierbaren Zuckern bei Temperaturen zwischen 20 und 40°C in an sich bekannter Weise, dadurch gekennzeichnet, daß bei pH-Werten zwischen 2,7 und 4,0 eine Hefe der Hinterlegungsnummer DSM 3649 gezüchtet wird und das gebildete Ethanol in an sich bekannter Weise abgetrennt wird.

2. Verfahren gemäß Anspruch 1, dadurch gekennzeichnet, daß kontinuierlich gearbeitet wird.

3. Verfahren gemäß Anspruch 1 oder 2, dadurch gekennzeichnet, daß bei Temperaturen von 30 bis 35°C gearbeitet wird.

4. Verfahren gemäß einem der Ansprüche 1 bis 3, dadurch gekennzeichnet, daß bei einem pH-Wert von 3,2 bis 3,8 gearbeitet wird.

5. Verfahren nach einem der Ansprüche 1 bis 4, dadurch gekennzeichnet, daß die Abtrennung des Ethanols durch Destillation erfolgt.

6. Verfahren nach einem der Ansprüche 1 bis 5, dadurch gekennzeichnet, daß als Rohstoffe stark infizierte und nicht pasteurisierte Rohstoffe eingesetzt werden.

7. Verfahren nach einem der Ansprüche 1 bis 6, dadurch gekennzeichnet, daß als Rohstoffe enzymatisch aufgeschlossene Stärken, Reststärken, Rübensäfte oder Melassen eingesetzt werden.

8. Hefe der Hinterlegungsnummer DSM 3649.

9. Verfahren zur Herstellung von Hefen der Hinterlegungsnummer DSM 3649, dadurch gekennzeichnet, daß enzymatisch aufgeschlossene Reststärke bei einem pH-Wert von 2,8 bis 2,5 bei Raumtemperatur gelagert wird, bis sich die säureunempfindliche Hefe der Hinterlegungsnummer DSM 3649 so stark vermehrt hat, daß alkoholische Gärung beobachtet wird und die sich dabei gebildete Hefe isolierbar wird.

10. Verwendung von Hefe der Hinterlegungsnummer DSM 3649 zur alkoholischen Gärung im pH-Bereich von 2,7 bis 4,0.